# EUROPEAN PATENT SPECIFICATION

(11) **EP 0 451 231 B1**
(45) Date of publication and mention of the grant of the patent: **25.10.1995**
(21) Application number: 90914639.1
(22) Date of filing: 24.09.1990
(51) Int. Cl.: G01T 1/166, G01T 1/164, A61B 6/04

(54) **A GANTRY AND PALLET ASSEMBLY USED IN NUCLEAR IMAGING**
GEHÄUSE- UND PALETTENANORDNUNG ZUR VERWENDUNG BEI NUKLEARER ABBILDUNG
ENSEMBLE DE PORTIQUE ET DE PALETTE UTILISE EN IMAGERIE NUCLEAIRE

(30) Priority: 27.10.1989 US 428188
(43) Date of publication of application: 16.10.1991
(73) Proprietor: TRIONIX RESEARCH LABORATORY, INCORPORATED, Twinsburg, OH 44087 (US)
(72) Inventor: LIM, Chun, Bin, Solon, OH 44139 (US); WALKER, Rick, W., Kent, OH 44240 (US); SOBIN, Robert, E., Cleveland, OH 44105 (US); PINKSTAFF, Carlos, D., Twinsburg, OH 44087 (US)
(74) Representative: W.P. Thompson & Co.
(86) International application number: US9005423
(87) International publication number: WO9106876

(56) References cited:
- US-A- 4 523 091
- US-A- 4 651 007
- US-A- 4 928 283

## Description

### BACKGROUND OF THE INVENTION

This invention relates to apparatus used in nuclear imaging and more particularly to a gantry and pallet assembly having at least one scintillation camera for conducting whole body or single photon emission computed tomography (SPECT) scans.

Medical technology has developed methods and apparatus which harness radiopharmaceuticals to provide images of a preselected region of the human body that has been made temporarily radioactive. The radiopharmaceutical introduced into the human body selectively accumulates within the region of interest. The radiation emitted by the radiopharmaceutical is detected by one or more scintillation cameras which are used to map the distribution of the radioactively tagged pharmaceuticals. The scintillation camera collects data representative of the radiation emitted by the radiopharmaceutical; the collected data is then used to reconstruct images corresponding to selected transverse planes through the region of interest as shown in U.S. Patent No. 4,057,727 issued to Muehllehner et al. on November 8, 1977.

Conventional images have an inherent limitation in that underlying and overlying radioactivity distributions are often superimposed upon the region of interest resulting in images having poor contrast. SPECT imaging eliminates this problem by obtaining multiple views about the patient and using appropriate computer software to generate images of the cross-sectional slices of the region of interest. This is accomplished by either continuously or incrementally rotating at least one scintillation camera around a patient while maintaining the camera close to the patient's body and substantially perpendicular to that region of the patient's body from which data is being collected. For example, as many as 360 views could be obtained if the scintillation camera was incrementally rotated one degree at a time to acquire a complete data set. It is common to rotate the cameras in increments of three degrees resulting in 120 acquisition points. Incremental rotation requires that the scintillation camera be held stationary at each incremental position while data collection takes place.

For more rapid data collection, the scintillation camera can be continuously rotated about the patient and data collected in separate images corresponding to preselected angular increments. This method is not as accurate as incremental rotation for detailed examinations.

When diagnostic rotational SPECT scanning is performed the patient lies on an arcuate narrow table, customarily known as a pallet, which conforms to the patient's body so the detectors can get as close to the patient as possible. Whole body scanning does not require camera head rotation but does require that the detector be translated over the patient's body in linear fashion. It is important that a detector passing above the patient lying on his or her back follows the contour of the patient's body thereby staying close to it. Because no rotation is required the patient lies on a pallet which is wider than the one used for SPECT scanning thereby providing more comfort to the patient. Moreover, the pallet should be flat so a detector passing underneath the patient can get as close to the patient's body as is needed for that particular scan.

Additionally, when conducting a rotational SPECT scan, the amount of time needed to conduct the scan decreases with the number of detectors used. Thus, it may be cost effective and easier on the patient to use two or three detector heads instead of just one when performing a scan.

Rotational SPECT imaging can be performed over any part of a patient's body. Thus, it is necessary to orient the scintillation camera or cameras relative to a pallet so they can be rotated around the region of interest. Additionally, present diagnostic procedures are such that it is economically and practically advantageous to utilize imaging apparatus capable of performing both rotational SPECT scanning and whole body scanning. Thus, the scintillation cameras must be able to translate the length of a patients body as well as rotate around it at any point along its axis.

In one commercially available system, the Siemens Rota Camera, a gantry is provided having a through hole of sufficient size to allow a patient supported on a pallet to pass at least part way therethrough. The through hole has a rotation ring mounted therein with two diametrically arranged detectors connected thereto a distance away from the rotation ring. The detectors are capable of being rotated in a plane perpendicular to the longitudinal axis of the through hole. The gantry has arranged therewith an automatically driven bed which can be longitudinally translated through the through hole to conduct a whole body scan. One disadvantage with this arrangement is that vibrations occur within the counter-balanced arms supporting the detectors during rotational SPECT scanning resulting in the acquisition of inaccurate data.

At least one other commercially available gantry, the Elscint Apex 415 ECT, employs concepts similar to the gantry just described including having the detectors mounted a distance away from the rotation ring. Another similar arrangement is disclosed in US-A-4,523,091, in which two diametrically opposed detectors are fixed to a ring which is rotatably mounted on a fixed gantry. The ring may be rotated to acquire data of a radioactively tagged pharmaceutical injected into a patients body; the means for rotating the ring permit the detectors to move so that they are in a horizontal plane. A patient table is movable and arranged substantially coaxial with the rotation axis. A common problem resulting from these designs is that vibrations occur during rotation due to the detectors being located such a distance from the rotation ring. Additionally, for the detectors to translate the length of a patient's body an overly long translation track must be provided.

In US-A-4,651,007, a single detector is translationally mounted on two horizontal rods whose ends are supported on rotatably mounted supports. The detector can be moved along the rods over a patient, and the orientation of the detector with respect to the supports can be adjusted.

Another commercially available nuclear imaging apparatus is disclosed in U.S. Patent No. 4,645,933 issued on February 24, 1987 to Gambini, et. al. Gambini discloses a medical diagnostic system using the principles of nuclear medicine including a gantry having a detector pivotally supported by a pair of parallel support arms which are pivotally mounted to a detector support section. The support arms are weighted at the end opposite the detector for tilting rotation of the detector about an axis parallel to the arm pivot axis. One disadvantage of this configuration is that it is difficult to obtain a precise orbital rotation of the detector around a patient during an ECT or rotational SPECT scan. Also, the length of the weighted arms contributes to the creation of vibrations during rotation or translation of the detector which can result in the acquisition of inaccurate data.

One manufacturer, ADAC, mitigated the effects of vibrations occurring during rotation or translation of the detector with their Genesys system by introducing a second ring coaxial with a first ring and rotationally mounting one detector therebetween. This approach reduces erroneous data acquisition by stemming vibrations but is limited in that it is not readily adaptable for use with more than one detector because of the location of the pallet upon which a patient lies during scanning. Furthermore, it is not practically possible to have a scan performed while standing or sitting. The location of the pallet inhibits performance of scans requiring the patient to be in a position other than supine.

In accordance with a first aspect of the present invention, there is provided a gantry and pallet assembly for use in nuclear imaging, comprising:
a gantry having a rotation ring defining an aperture through which a patient may pass during a scan, said rotation ring also defining a longitudinal axis when laterally translated;
two diametrically disposed detectors rigidly connected to said ring;
means for radially translating the detectors on the ring so that the detectors may move radially relative to a patient and the longitudinal axis;
means for rotating said ring so that said detectors are rotated thereby acquiring data used for mapping the distribution of a radioactively tagged pharmaceutical previously injected into a patient's body, the means for rotating said ring having at least one position in which the disposed detectors are in a horizontal plane;
a first pallet supported at each end by a supporting means so that said first pallet is substantially coaxial with said longitudinal axis, said pallet being positioned so that it is easily accessible to a patient getting onto or off of it;
means for laterally translating said gantry relative to said first pallet along a guide means so that said detectors pass over the patient's body supported on said first pallet thereby acquiring data from diametrical areas of the patient's body used for mapping the distribution of radioactively tagged pharmaceutical previously injected into the patient's body, the patient's body passing at least partially within said aperture when said gantry and said first pallet are translated relative to each other.

In accordance with a second aspect of the present invention, there is provided a gantry assembly for use in nuclear imaging, comprising:
a gantry having a rotation ring rotationally disposed thereon, said rotation ring defining an aperture through which a patient may pass during a scan;
a detector connected in a generally rigid manner to said rotation ring;
means for rotating said rotation ring so that said detector is rotated thereby acquiring data used for mapping the distribution of a radioactively tagged pharmaceutical previously injected into a patient's body, the means for rotating said ring having at least one position wherein the detector is in a horizontal facing position;
means for laterally translating said gantry relative to a pallet along a guide means for supporting a patient, whereby said detector passes over the patient's body acquiring data from diametrical areas of the patient's body used in conducting a whole body scan;
means for radially translating said detector relative to said ring so that said detector can be moved relative to the patient thereby providing optimum positioning of said detector during performance of a scan.

Accordingly, the present invention provides a gantry having two diametrically disposed detectors mounted on and in close proximity to a single rotation ring. The rotation ring can be rotated around a patient thereby acquiring data used for rotational SPECT scanning. The ring can also be laterally translated thereby conducting a whole body scan. A pallet for each type of scan is provided upon which a patient lies. The pallet is supportable at each end by a supporting means and is positioned so that it passes through the rotation ring when translated. The position of the pallet makes it easily accessible for a patient getting onto or off of it.

Also disclosed herein, but not part of the invention as defined in the claims, is a substantially rectangular pallet used for whole body scanning and a pallet assembly for storing and moving the pallet. The pallet assembly has means for supporting the pallet at each end to immobilize it during scans. The pallet assembly also has a storage carriage made of a base having a frame pivotally mounted a distance above it. The frame has an extendable section upon which the pallet is supported. The storage carriage can be moved between a position close to the gantry and a position away from the gantry where it won't interfere with scanning procedures. When the storage carriage is close to the gantry the extandable section of the frame can be moved to a position where the pallet is over the pallet supporting means.

It is still a feature of the invention to provide a means for radially translating the diametrically disposed detectors relative to the rotation ring. This allows the detectors to be maintained the optimum distance away from the patient while a rotational SPECT scan is being conducted. The detectors may be radially translated simultaneously with their rotation. For example, when a patient is lying on his or her back the patient's body width is greater than his or her body height. As the detectors are rotated their radial motion allows them to follow the contours of the patient's body thereby acquiring the most accurate data.

The scintillation cameras used with the invention are of a type commercially available such as that disclosed in U.S. Patent No. 4,523,091 issued on June 11, 1985 to Persky.

The gantry and pallet assembly are configured so that if a particular imaging procedure does not require a patient to be supine the pallet can be removed from the support devices and the detectors oriented to a position where the patient could stand or sit between them. This procedure cannot be adopted for rotational SPECT scanning but it is commonly used for spot scanning where the rotation ring has one or two detectors rigidly connected thereto.

### BRIEF DESCRIPTION OF THE DRAWINGS

FIG. 1 is a perspective view of the gantry and that part of the pallet assembly used for supporting and transferring the rectangular pallet; the rectangular pallet is shown in position for scanning to be conducted;
FIG. 1A is a view of FIG. 1 with the frame of the pallet assembly in its fully extended position;
FIG. 2 is a view of FIG. 1 with the gantry translated a distance along the translation tracks;
FIG. 3 is a view of FIG. 1 with the rectangular pallet in its storage position and the arcuate pallet in position for scanning;
FIG. 4 is a side view of the gantry and pallet assembly with a patient supine on a pallet;
FIG. 5 is a front view of the gantry with a patient standing in position for a scan not requiring the patient to be supine.
FIG. 6 is an exploded view of the means for rotating the rotation ring, means for translating the gantry, and means for radially translating the detectors;
FIG. 7A is an end view of the pallet assembly with the arcuate pallet rack assembly in an extended position; and
FIG. 7B is an end view of the pallet assembly with the arcuate pallet rack assembly in a retracted position.

### DETAILED DESCRIPTION OF THE PREPERRED EMBODIMENT

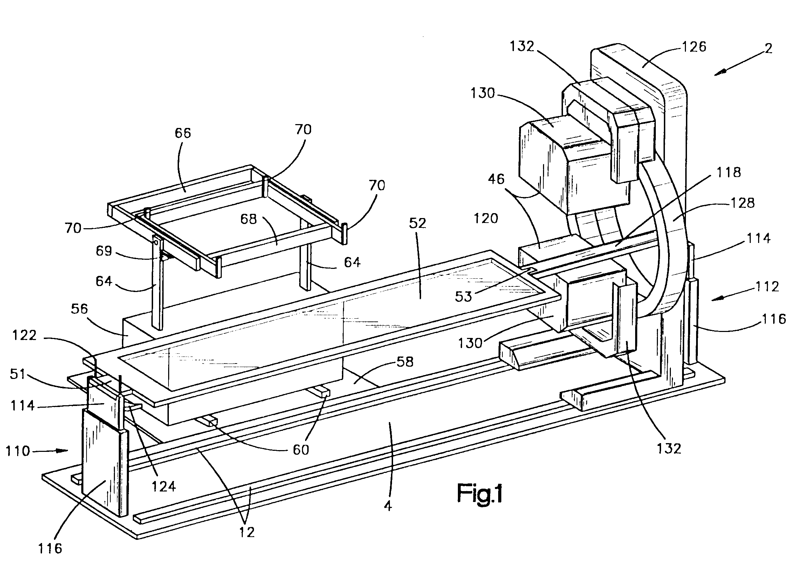

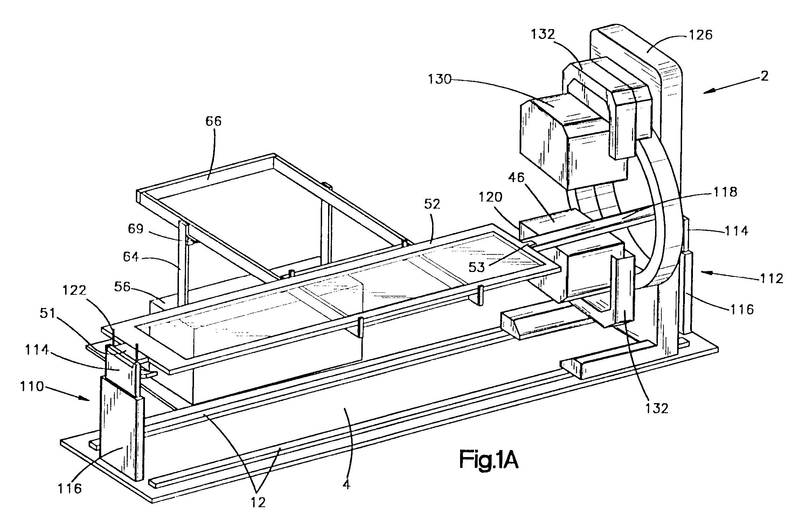

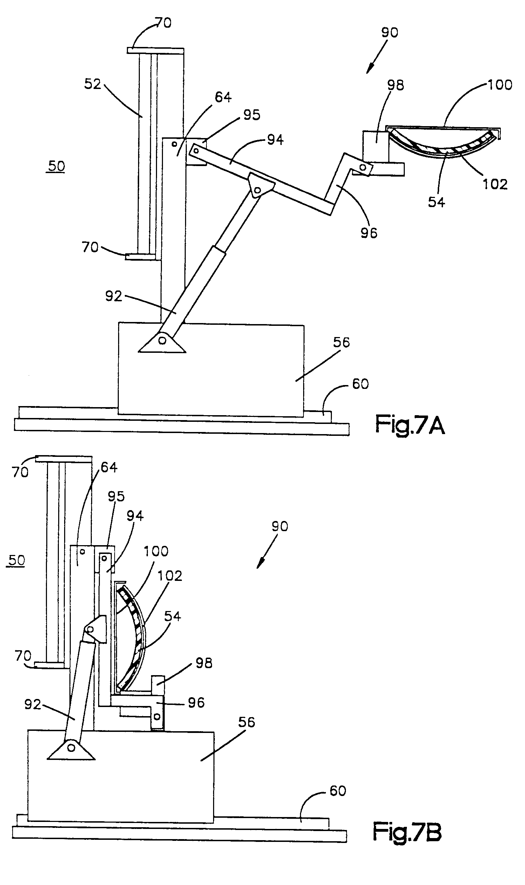

Referring now to the drawings and in particular to Fig. 1, there is shown the gantry 2 and that part of the pallet assembly used for storing and transferring the pallet 52. The pallet assembly includes the storage carriage 50, the front pallet support 110 and the rear pallet support 112. The storage carriage 50 is shown in Figs. 7A and 7B to also include an arcuate pallet rack assembly generally referred to by reference numeral 90. Fig. 7A shows the rack assembly 90 in an extended position and Fig. 7B shows it in a retracted position. The operation of the rack assembly 90 will be more fully described hereinafter.

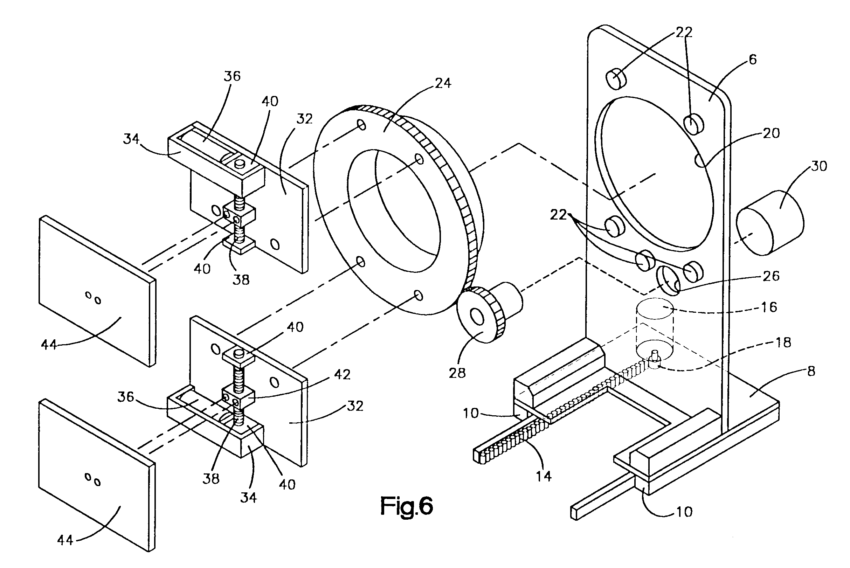

Referring to Figs. 1 and 6, the structure of the gantry 2 is clearly shown positioned on the gantry support platform 4. An upright gantry plate 6 is rigidly connected perpendicular to the gantry base 8 in any conventional manner. The gantry base 8 has a pair of support legs 10 which cooperate with the translation tracks 12 which are connected in a substantially parallel manner to the support platform 4 as shown in Fig. 1. The support legs 10 also act as a cantilevered base which helps the gantry 2 maintain its balance.

The gantry 2 is laterally translated along the support platform 4 by a chain and sprocket arrangement as shown in Fig. 6. Drive chain 14 is connected at each end to the support platform 4 in a taut manner. A driving motor 16 rotationally cooperates with a sprocket 18. The sprocket 18 interacts with the drive chain 14 so that when the driving motor 16 is activated the gantry 2 is moved along the translation tracks 12. The gantry 2 can be translated in either direction along the translation tracks 12.

It is apparent that other arrangements could be utilized to translate the gantry 2. For example, a worm gear spanning the length of the support platform 4 and having a block movingly disposed thereon could be used. The worm gear would be rotationally journalled at each end and the block connected to the gantry 2. When the worm gear is mechanically rotated the block and gantry 2 are translated in either direction along the translation tracks 12. Alternatively, other known arrangements could be used to laterally translate the gantry 2.

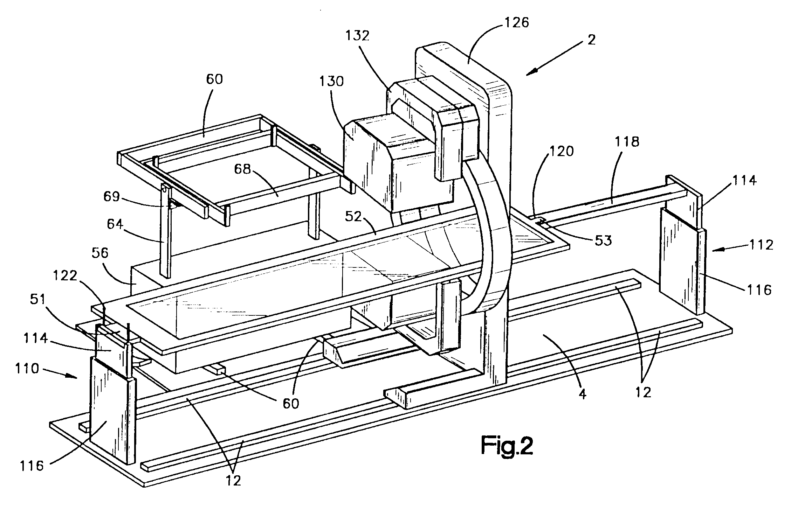

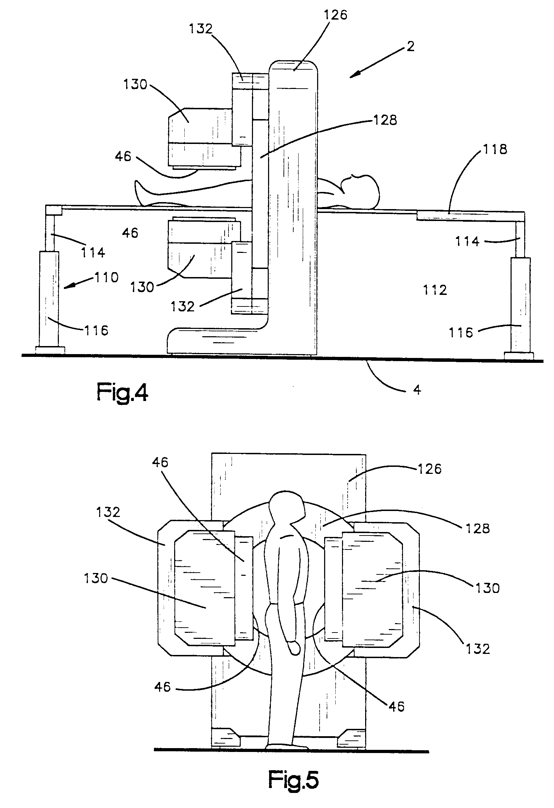

Referring to Fig. 6, the gantry plate 6 has an aperture 20 disposed therein of sufficient diameter so that a patient supported on a pallet can pass longitudinally therethrough. Fig. 4 shows the gantry 2 translated a distance along support platform 4. As the gantry 2 is translated the supine patient supported on the pallet 54 will pass through the aperture 20. The gantry 2 can then be stopped at any point along the axis of the patient's body to conduct the appropriate scan. Fig. 2 also shows the gantry 2 translated a distance along the translation tracks 12. The storage carriage 50 is moved away from the platform 4 so the gantry 2 has ample room to be translated.

The gantry plate 6 has five groove track rollers 22 rigidly connected thereto and configured around the aperture 20 as shown in Fig. 6. The groove track rollers 22 are adapted to rotationally support a rotation ring 24 in any conventional manner. The gantry plate 6 has further disposed therein a spur gear mounting slot 26 into which a spur gear 28 is inserted. The spur gear 28 rotationally cooperates with the rotation ring 24 when both are mounted in position on the gantry plate 6. The spur gear 28 is drivingly connected to a drive motor 30 in a conventional manner so that when the drive motor 30 is activated the rotation of spur gear 28 causes the rotation ring 24 to rotate. It is apparent that other gearing arrangements could be adopted to impart rotation upon the rotation ring 24 which in turn rotates the detectors 46. Other gearing arrangements may utilize alternative approaches without deviating from the scope of the invention.

The rotation ring 24 has two radial back plates 32 rigidly connected thereto in a diametrically opposed manner as shown in Fig. 6. The back plates 32 can be connected to the rotation ring 24 in a known manner, such as nuts and bolts, or they could be permanently anchored to the rotation ring 24. It is preferred they are connected in a removable fashion for maintenance and adjustment purposes. In the preferred embodiment, each back plate 32 has connected thereto a motor mount 34 adapted to contain a drive motor 36. Each drive motor 36 is mechanically coupled in a known manner to a worm gear 38 journalled at each end to worm gear supports 40 which are rigidly connected to the radial back plate 32. Each worm gear 38 has movingly disposed thereon a mounting block 42 to which a detector mounting plate 44 is solidly mounted in a conventional manner. When the drive motors 36 are activated they mechanically drive the worm gears 38 thereby causing the mounting blocks 42 to be radially translated in either direction relative to the rotation ring 24. When the mounting blocks 42 are radially translated the detectors 46 are translated therewith via their coupling to the mounting blocks 42. The drive motor 30 and the drive motors 36 can be simultaneously activated thereby radially translating the detectors 46 simultaneously with the rotation of rotation ring 24. This dual action movement provides optimum results when conducting a rotation SPECT scan.

Referring now to Fig. 1, there is shown a storage carriage, generally referred to by reference numeral 50, which is adapted to support a substantially flat pallet 52 used for whole body scaning and an arcuate pallet 54 used for rotation SPECT scanning. It is important the pallet 52 used for whole body scanning be flat so the detectors can get close to the patients body. The preferred embodiment of the present invention provides a flat pallet which is substantially rectangular. It is apparent that other shapes could be provided without deviating from the scope of the invention.

The storage carriage 50 has a substantially rectangular base 56 movably mounted on a support skid 58. The support skid 58 is substantially perpendicular to the gantry support platform 4 and may be connected to it. The support skid 58 has connected thereto a pair of substantially parallel guide rails 60 that interact with the bottom of the base 56. Thus, the storage carriage 50 can be displaced between a position close to the gantry support platform 4 and a position spaced away from it. When the storage carriage 50 is spaced away from the platform 4 it will not interfere with scanning procedures. The storage carriage 50 may ride on wheels or non-rolling guide means.

Certain scanning procedures are best conducted with the patient standing or sitting. Fig. 5 shows a patient standing with the detectors 46 oriented to conduct a scan. The preferred embodiment of the present invention allows a patient to easily access the detectors 46 in this manner. The patient may stand as shown or sit down if necessary.

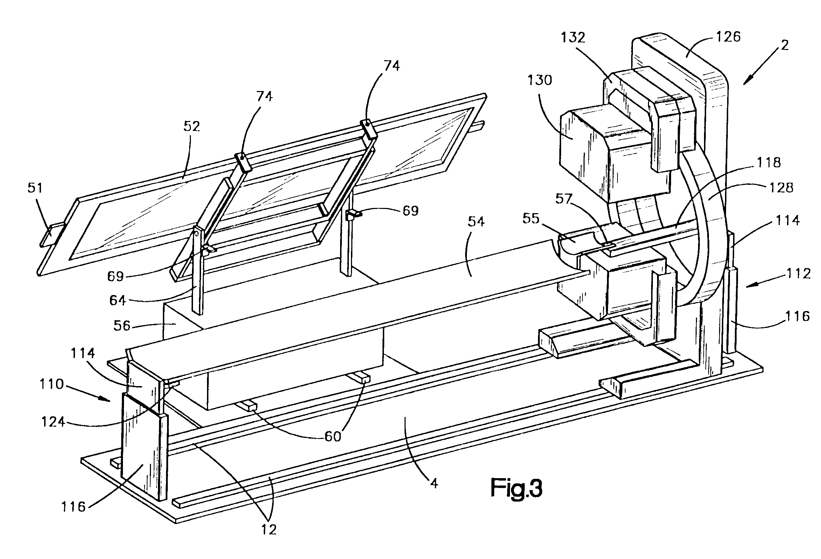

Referring again to Fig. 1, the base 56 has perpendicularly extending therefrom and connected thereto a pair of vertical frame support posts 64 which pivotally support a frame having an external frame component 66 and an extendable frame component 68. The extendable frame component 68 is slidably contained inside the external frame component 66 so that the extendable component 68 can be moved between an extended position for transferring the rectangular pallet 52, as shown in Fig. 1A, and a retracted position for storing it, as shown in Fig 3. Each support post 64 has connected thereto a cantilevered buttress 69 which prevents the frame from rotating past horizontal.

The extendable frame component 68 has four pallet retaining pegs 70 extending perpendicularly therefrom and firmly connected thereto. The pegs 70 help guide the pallet 52 into place when being placed on the extendable frame component 68.

Referring to Fig. 1A, to transfer the pallet 52 from the vertically adjustable support means 110, 112 to the storage carriage 50, the pallet 52 is raised so that the extendable frame component 68 can be extended underneath it when the storage carriage 50 is moved close to the platform 4. The support means 110, 112 then lower the pallet 52 onto the extendable frame component 68. The extendable frame component 68 can then be retracted into its storage position as best shown in Fig. 3. The storage carriage 50 can then be moved away from the platform 4 so that other scans can be performed.

The frontmost pegs on the extendable frame component 68 have apertures therein into which a spring loaded detent 74 is inserted to exert pressure against the edge of the pallet 52. Thus, the pallet 52 can be tilted into its storage position which is close to vertical as shown in Fig. 3.

Referring now to Figs. 7A and 7B, there is shown the storage carriage 50 with an arcuate pallet rack assembly generally referred by reference numeral 90. The rack assembly 90 has a pair of telescoping arms 92 pivotally connected at one end to the base 56. The arms 92 can telescope in any conventional manner. Each arm 92 has pivotally connected near its other end a cross member 94. Each cross member 94 has an L-shaped extension rigidly connected thereto as best seen in Fig. 7A. The other end of each cross member 94 is pivotally connected to a brace 95 mounted on the frame support posts 64. A hinged pallet coupling 98 is pivotally connected to each L-shaped extension 96. The couplings 98 have connected thereto a retaining bracket 100 and a pallet support brace 102. The retaining brackets 100 and the support braces 102 are adapted to firmly grasp therebetween the arcuate pallet 54 for storing and transferring as shown in Figs. 7A and 7B.

The rack assembly 90 is used to displace the arcuate pallet 54 between a storage position shown in Fig. 7B, and a transfer position as shown in Fig. 7A. When the pallet 54 is in the transfer position the retaining brackets 100 are pivoted so that the pallet 54 can be lifted off the pallet support braces 102. Thus, the pallet 54 can be transferred onto the pallet support means 110, 112 for rotational SPECT scanning.

Referring now to Fig. 1, 1A, and 3 either pallet 52, 54 can be detachably supported on front pallet support 110 and rear pallet support 112 for use when scans are being performed. Both the front and rear pallet supports 110, 112 are vertically adjustable so that a pallet placed thereon can be moved up and down thereby achieving the proper spatial relationship between a patient and the detectors 46 when conducting a scan. Each pallet support 110, 112 has a vertically extendable component 114 which is movably contained within a pallet support housing 116. The extendable components 114 can be controlled in any conventional manner so they telescope relative to the support housings 116. The preferred method of control is a worm gear arrangement which provides for precise adjustments.

The rear pallet support 112 has a cantilevered arm 118 extending perpendicularly therefrom. The cantilevered arm 118 has a notch 120 formed therein into which one end of either pallet is detachably supported. The pallet 52 has a support peg 53 connected to one end which firmly fits within the notch 120. The other end of the pallet 52 has a support flange 51. The support flange 51 cooperates with a retaining bar 122 which is mounted on the vertically extendable component 114 of the front pallet support 110. When the support flange 51 is placed within the retaining bar 122, and the support peg 53 is placed within the notch 120, the pallet 52 is firmly held in place for scanning purposes.

Similarly, the arcuate pallet 54 has connected at one end a head rest 55. The head rest 55 has connected thereto a support peg 57. The support peg 57 firmly fits within the notch 120. The other end of the pallet 54 rests upon an arcuate ledge 124 connected to the vertically extendable component 114 of the front pallet support 110. When the support peg 57 is placed within the notch 120, and the other end of the pallet 54 is placed upon the arcuate ledge 124, the pallet 54 is firmly held in place for scanning purposes as best shown in Fig. 3.

The internal structure of the gantry 2 is protected by a gantry plate cover 126 as are other components of the preferred embodiment of the invention. The rotation ring 24 has a cover 128 placed thereover which snugly cooperates with the cover 126. The detectors 46 have covers 130 protecting them and covers 132 are placed over the mechanisms which radially translate the detectors. The covers 126, 128, 130, and 132 prevent dirt and other particles from fouling the operations of the machinery and are aesthetically appealing.

While the preferred embodiment of the invention has been shown and described in detail, it is recognized that various modifications and rearrangements may be resorted to without departing from the scope of the invention as defined in the claims.

## Claims

1. A gantry and pallet assembly for use in nuclear imaging, comprising:
a gantry (2) having a rotation ring (24) defining an aperture through which a patient may pass during a scan, said rotation ring also defining a longitudinal axis when laterally translated;
two diametrically disposed detectors (46) rigidly connected to and in close proximity to said ring;
means for radially translating the detectors (46) on the ring (24) so that the detectors may move radially relative to a patient and the longitudinal axis;
means (30) for rotating said ring so that said detectors are rotated thereby acquiring data used for mapping the distribution of a radioactively tagged pharmaceutical previously injected into a patient's body, the means for rotating said ring having at least one position in which the disposed detectors are in a horizontal plane;
a first pallet (52) supported at each end by a supporting means (110,112) so that said first pallet is substantially coaxial with said longitudinal axis, said pallet being positioned so that it is easily accessible to a patient getting onto or off of it;
means (14,16) for laterally translating said gantry (2) relative to said first pallet (52) along a guide means so that said detectors (46) pass over the patient's body supported on said first pallet (52) thereby acquiring data from diametrical areas of the patient's body used for mapping the distribution of radioactively tagged pharmaceutical previously injected into the patient's body, the patient's body passing at least partially within said aperture when said gantry and said first pallet are translated relative to each other.

2. A gantry and pallet assembly for use in nuclear imaging as claimed in claim 1, wherein the first pallet (52) is positioned so that one end is adjacent at least one detector (46) when said gantry is in one position so that said at least one detector can acquire data from a patient's head region when lying on said pallet, the data being used for mapping the distribution of a radioactively tagged pharmaceutical previouusly injected into the patient's body.

3. A gantry and pallet assembly for use in nuclear imaging as claimed in claim 2, wherein said supporting means (110,112) is vertically adjustable so that the spatial relationship of said pallet (52) to said at least one detector (46) can be adjusted thereby moving a patient supported by said pallet closer to or further away from said at least one detector to achieve the desired positioning of the patient to perform a particular imaging procedure, said rotation ring (24) being capable of being incrementally rotated.

4. A gantry and pallet assembly for use in nuclear imaging as claimed in any of claims 1 to 3, wherein said first pallet (52) is substantially rectangular and flat and used for whole body scanning whereby in use said gantry (2) is laterally translated relative to said first pallet (52) along a guide means (12) causing said first pallet to pass longitudinally through said aperture, said first pallet being detachably supported at each end to said supporting means.

5. A gantry and pallet assembly for use in nuclear imaging as claimed in claim 4, wherein said first pallet (52) is interchangeable with a second pallet (54) which is substantially arcuate and used for rotational SPECT scanning whereby said at least one detector is positioned over an area of interest of the patient's body then rotated thereby acquiring a sufficient amount of data to map the distribution of a radioactively tagged pharmaceutical previously introduced into the patient's body, said second pallet (54) being detachable supported by said supporting means (110,112), said first and said second pallets being storable on a storage carriage (50) positioned in close proximity to said gantry (2), said storage carriage having a frame (64) mounted above a base (56) and adapted to retain said first pallet, said frame having an extendable frame component (68) which can be positioned so that said first pallet can be easily removed therefrom or placed thereon.

6. A gantry and pallet assembly for use in nuclear imaging as claimed in claim 5, wherein said storage carriage (50) further comprises a pair of frame support posts (64) extending from said base (56), said extendable frame component (68) being pivotally mounted to said posts, each of said posts having a cantilever buttress connected thereto for preventing said frame from pivoting past horizontal in one direction, said extendable frame component having four pallet retaining pegs (70) extending therefrom for retaining said first pallet (52), two of said pegs each having an aperture therein into which a spring loaded detent (74) is inserted for exerting pressure against said first pallet when positioned on said frame thereby holding said first pallet firmly within said frame.

7. A gantry and pallet assembly for use in nuclear imaging as claimed in claim 5 or claim 6, wherein said extendable frame component (68) is slidably mounted inside an external frame component so that said extendable frame component can be positioned between a storage position and a position where said first pallet (52) can be easily removed therefrom or placed thereon, said storage carriage being movably positioned substantially parallel to said longitudinal axis so that said storage carriage (50) can be positioned close to said gantry (2) for transferring said pallets from said storage carriage to said supporting means (110,112) or from said supporting means to said storage carriage, or away from said gantry so it does not interfere with the scanning procedures.

8. A gantry and pallet assembly for use in nuclear imaging as claimed in any of claims 5 to 7, further comprising means (92) for displacing said arcuate pallet (54) between a retracted position and a position where said arcuate pallet can be easily transferred to or from said support means.

9. A gantry and pallet assembly for use in nuclear imaging as claimed in claim 8, wherein said displacing means comprises a pair of telescoping arms (92) pivotally connected to said base (56) in a substantially parallel manner, said telescoping arms having connected thereto means (98) for grasping said arcuate pallet and moving it between a position where said pallet can be easily transferred to or from said support means, and a position where said pallet is stored in close proximity to said frame support posts (64).

10. A gantry and pallet assembly for use in nuclear imaging as claimed in any of claims 4 to 9, wherein said guide means (12) comprises a pair of substantially parallel translation tracks mounted on a gantry support platform (4) so that said gantry can be translated along said translation tracks.

11. A gantry and pallet assembly for use in nuclear imaging as claimed in any of the preceding claims, wherein said gantry (2) comprises a gantry plate (6) having a second aperture (20) disposed therein so that said pallet (52) can pass through said second aperture when said gantry is translationally moved relative to said pallet, said gantry plate (6) being perpendicularly connected to a gantry base (8) having at least one support leg (10) extending therefrom, said rotation ring (24) being rotationally disposed on said gantry plate coaxial with said aperture.

12. A gantry and pallet assembly for use in nuclear imaging as claimed in claim 11, wherein said gantry plate (6) has perpendicularly connected thereto a plurality of groove track rollers (22) adapted to cooperate with said rotation ring (24) for rotationally guiding said rotation ring, said means (30) for rotating said rotation ring comprising a first driving motor (30) rotationally connected to a spur gear (28) which rotationally cooperates with said rotation ring, said spur gear being supported in a slot (26) disposed in said gantry plate.

13. A gantry and pallet assembly for use in nuclear imaging as claimed in any of the preceding claims, further comprising a pair of radial back plates (32) connected to said rotation ring (24), each of said back plates having movingly connected thereto a detector mounting plate (44), said means for radially translating said detectors comprising a worm gear (38) journalled at each end to a worm gear support (40) connected to said back plate (32), said worm gear having a mounting block (42) movingly disposed thereon to which said detector mounting plate (44) is connected, and a driving motor (36) for cooperating with said worm gear so that said detectors are radially translated relative to said ring.

14. A gantry and pallet assembly for use in nuclear imaging as claimed in claim 13, wherein each of said back plates (32) further comprises a motor mount (34) to which said driving motor is mounted, said means for translating each of said detectors being disposed between said radial back plate (32) and said detector mounting plate (44).

15. A gantry and pallet assembly for use in nuclear imaging as claimed in any of the preceding claims, wherein said means for producing relative translational movement between said gantry and said pallet comprises a second driving motor (16) having a sprocket (18) rotationally connected thereto, said driving motor and sprocket connected to said gantry base (8), a drive chain (14) which engagingly cooperates with said sprocket (18), said drive chain connected at each end to said support platform so that when said second driving motor is activated said sprocket cooperates with said driving chain thereby translating said gantry along a pair of translation tracks (12) which are substantially parallel to a longitudinal axis of said rotation ring.

16. A gantry and pallet assembly for use in nuclear imaging as claimed in any of the preceding claims, wherein the means for producing relative translational movement between the gantry and the pallet comprises means for laterally translating the gantry along a translational track (12) supported along its length.

17. A gantry assembly for use in nuclear imaging, comprising:
a gantry (2) having a rotation ring (24) rotationally disposed thereon, said rotation ring defining an aperture through which a patient may pass during a scan;
at least one detector (46) connected in a generally rigid manner and in close proximity to said rotation ring;
means (30) for rotating said rotation ring (24) so that said at least one detector (46) is rotated thereby acquiring data used for mapping the distribution of a radioactively tagged pharmaceutical previously injected into a patient's body, the means for rotating said ring having at least one position wherein the detector is in a horizontal facing position;
means (14,16) for laterally translating said gantry (2) relative to a pallet (52,54) along a guide means for supporting a patient, whereby said at least one detector (46) passes over the patient's body acquiring data from diametrical areas of the patient's body used in conducting a whole body scan;
means (38) for radially translating said at least one detector (46) relative to said ring so that said detector can be moved relative to the patient thereby providing optimum positioning of said detector during performance of a scan.

18. A gantry assembly for use in nuclear imaging as claimed in claim 17, wherein a pair of substantially parallel translation tracks (12) are mounted on a gantry support platform (4) so that said gantry can be translated along said translation tacks, said rotation ring being capable of being incrementally rotated.

19. A gantry assembly for use in nuclear imaging as claimed in claim 17 or claim 18, comprising two opposed detectors and wherein said gantry (2) comprises a gantry plate (6) having a second aperture (20) disposed therein so that the pallet can pass through said second aperture when said gantry is translationally moved relative to said pallet, said gantry plate being perpendicularly connected to a gantry base (8) having at least one support leg (10) extending therefrom, said rotation ring being rotationally disposed on said gantry plate coaxial with said aperture.

20. A gantry assembly for use in nuclear imaging as claimed in claim 19, wherein said gantry plate (6) has perpendicularly connected thereto a plurality of groove track rollers (22) adapted to cooperate with said rotation ring for rotationally guiding said ring.

21. A gantry assembly for use in nuclear imaging as claimed in claim 19 or claim 20, wherein said means for rotating said rotation ring (24) comprises a first driving motor (30) rotationally connected to a spur gear (28) which rotationally cooperates with said rotation ring, said spur gear being supported in a slot (26) disposed in said gantry plate.

22. A gantry assembly for use in nuclear imaging as claimed in any of claims 19 to 21, further comprising a pair of radial back plates (32) connected to said rotation ring, each of said back plates having movingly connected thereto a detector mounting plate (44), said means for radially translating each of said detectors comprising a worm gear (38) journalled at each end to a worm gear support (40) connected to said back plate (32), said worm gear having a mounting block (42) movingly disposed thereon to which a detector mounting plate is connected, and a driving motor (36) for cooperating with said worm gear so that said detectors may be radially translated relative to said ring.

23. A gantry assembly for use in nuclear imaging as claimed in claim 22, wherein each of said back plates (32) further comprises a motor mount (34) to which said driving motor (36) is mounted, said means for translating each of said detectors being disposed between a radial back plate and a detector mounting plate.

24. A gantry assembly for use in nuclear imaging as claimed in any of claims 17 to 23, wherein said means for laterally translating said gantry comprises a second driving motor (16) having a sprocket (18) rotationally connected thereto, said driving motor and sprocket being connected to said gantry base (8), a drive chain (14) which engagingly cooperates with said sprocket, said drive chain connected at each end to said support platform so that when said second driving motor is activated said sprocket cooperates with said driving chain thereby translating said gantry along a pair of translation tracks (12) which are substantially parallel to a longitudinal axis of said rotation ring.

25. A gantry assembly for use in nuclear imaging as claimed in any of claims 17 to 24, wherein the means for producing relative translational movement between the gantry and the pallet comprises means for laterally translating the gantry along a translational track (12).

## Patentansprüche

1. Gehäuse- und Palettenanordnung zur Verwendung bei nuklearer Abbildung, die folgendes umfaßt:
ein Gehäuse (2) mit einem Rotationsring (24), der eine Öffnung definiert, durch die ein Patient während eines Scans passieren kann, wobei der Rotationsring bei einer lateralen Verschiebung auch eine Längsachse definiert;
zwei diametrisch angeordnete Detektoren (46), die sehr nahe an dem genannten Ring fest mit diesem verbunden sind;
ein Mittel zur radialen Verschiebung der Detektoren (46) auf dem Ring (24), so daß sich die Detektoren radial relativ zu einem Patienten und der Längsachse bewegen können;
ein Mittel (30) zum Drehen des genannten Ringes, so daß die genannten Detektoren gedreht werden, wodurch Daten zur Darstellung der Verteilung eines radioaktiv markierten Pharmazeutikums erfaßt werden, das zuvor in den Körper eines Patienten eingespritzt wurde, wobei das Mittel zum Drehen des genannten Ringes wenigstens eine Stellung aufweist, in der sich die angeordneten Detektoren in einer horizontalen Ebene befinden;
eine erste Palette (52), die an beiden Enden durch ein Tragmittel (110, 112) getragen werden, so daß die genannte erste Palette im wesentlichen koaxial mit der genannten Längsachse ist, wobei die genannte Palette so positioniert ist, daß sie für einen Patienten zum Besteigen oder Verlassen leicht zugängig ist;
ein Mittel (14, 16) zum lateralen Verschieben des genannten Gehäuses (2) relativ zu der genannten ersten Palette (52) entlang eines Führungsmittels, so daß die genannten Detektoren (46) über den Körper des Patienten passieren, der auf der genannten ersten Palette (52) ruht, wodurch Daten von diametrischen Bereichen des Körpers des Patienten erfaßt werden, die zur Darstellung der Verteilung eines radioaktiv markierten Pharmazeutikums benutzt werden, das zuvor in den Körper des Patienten eingespritzt wurde, wobei der Körper des Patienten wenigstens teilweise durch die genannte Öffnung passiert, wenn das genannte Gehäuse und die genannte erste Palette relativ zueinander verschoben werden.

2. Gehäuse- und Palettenanordnung zur Verwendung bei nuklearer Abbildung nach Anspruch 1, wobei die erste Palette (52) so positioniert ist, daß sich ein Ende neben wenigstens einem Detektor (46) befindet, wenn sich das genannte Gehäuse in einer Stellung befindet, so daß der genannte wenigstens eine Detektor Daten vom Kopfbereich eines Patienten erfassen kann, wenn dieser auf der genannten Palette liegt, wobei die Daten zur Darstellung der Verteilung eines radioaktiv markierten Pharmazeutikums benutzt werden, das zuvor in den Körper des Patienten eingespritzt wurde.

3. Gehäuse- und Palettenanordnung zur Verwendung bei nuklearer Abbildung nach Anspruch 2, wobei das genannte Tragmittel (110, 112) senkrecht verstellbar ist, so daß die räumliche Beziehung der genannten Palette (52) zu dem genannten wenigstens einen Detektor (46) verstellt werden kann, so daß ein auf der genannten Palette liegender Patient näher zu dem genannten einen Detektor hin oder weiter von diesem weg bewegt werden kann, um die zur Durchführung eines bestimmten Abbildungsverfahrens gewünschte Positionierung des Patienten zu erzielen, wobei der genannte Rotationsring (24) schrittweise gedreht werden kann.

4. Gehäuse- und Palettenanordnung zur Verwendung bei nuklearer Abbildung nach einem der Ansprüche 1 bis 3, wobei die genannte erste Palette (52) im wesentlichen rechteckig und flach ist und für einen Ganzkörperscan benutzt wird, so daß bei Gebrauch das genannte Gehäuse (2) lateral relativ zu der genannten ersten Palette (52) über ein Führungsmittel (12) verschoben wird, so daß die genannte erste Palette in Längsrichtung durch die genannte Öffnung passiert, wobei die genannte erste Palette lösbar an jedem Ende des genannten Tragmittels gelagert ist.

5. Gehäuse- und Palettenanordnung zur Verwendung bei nuklearer Abbildung nach Anspruch 4, wobei die genannte erste Palette (52) gegen eine zweite Palette (54) ausgetauscht werden kann, die im wesentlichen bogenförmig ist und für einen rotierenden SPECT-Scan benutzt wird, bei dem der genannte wenigstens eine Detektor über einen bestimmten Bereich des Körpers des Patienten positioniert und dann gedreht werden kann, um so eine ausreichende Menge an Daten zu erfassen, um die Verteilung eines radioaktiv markierten Pharmazeutikums darzustellen, das zuvor in den Körper des Patienten eingeführt wurde, wobei die genannte zweite Palette (54) lösbar auf dem genannten Tragmittel (110, 112) gelagert ist, wobei die genannte erste und die genannte zweite Palette auf einem Lagerungsschlitten (50) gelagert werden können, der sich in unmittelbarer Nähe zu dem genannten Gehäuse (2) befindet, wobei der genannte Lagerungsschlitten ein auf einer Basis (56) montiertes Gestell (64) aufweist und die genannte erste Palette hält, wobei das genannte Gestell eine ausziehbare Gestellkomponente (68) aufweist, die so positioniert werden kann, daß die genannte erste Palette leicht davon enffernt oder auf sie gesetzt werden kann.

6. Gehäuse- und Palettenanordnung zur Verwendung bei nuklearer Abbildung nach Anspruch 5, wobei der genannte Lagerungsschlitten (50) weiterhin ein Paar Gestellständer (64) aufweist, die von der genannten Basis (56) verlaufen, wobei die genannte ausziehbare Gestellkomponente (68) schwenkbar an den genannten Ständern montiert ist, wobei mit jedem der genannten Ständer eine Widerlagerstütze verbunden ist, um zu verhindern, daß das genannte Gestell in einer Richtung über die Horizontale hinaus schwenkt, wobei die genannte ausziehbare Gestellkomponente vier Palettenhaltestifte (70) aufweist, die zum Halten der genannten ersten Palette (52) von dieser weg verlaufen, wobei zwei der genannten Stifte jeweils eine Öffnung darin aufweisen, in die eine gefederte Arretierung (74) eingesetzt wird, die Druck auf die genannte erste Palette ausübt, wenn diese sich in dem genannten Gestell befindet, wodurch die genannte erste Palette in dem genannten Gestell festgehalten wird.

7. Gehäuse- und Palettenanordnung zur Verwendung bei nuklearer Abbildung nach Anspruch 5 oder Anspruch 6, wobei die genannte ausziehbare Gestellkomponente (68) verschiebbar in einer externen Gestellkomponente montiert ist, so daß die genannte ausziehbare Gestellkomponente zwischen einer Lagerungsstellung und einer Stellung positioniert werden kann, in der die genannte erste Palette (52) leicht von dem Gestell genommen oder auf das Gestell gelegt werden kann, wobei der genannte Lagerungsschlitten beweglich im wesentlichen parallel zu der genannten Längsachse positioniert ist, so daß der genannte Lagerungsschlitten (50) in der Nähe des genannten Gehäuses (2) positioniert werden kann, um die genannten Paletten von dem genannten Lagerungsschlitten zu dem genannten Tragmittel (110, 112) oder von dem genannten Tragmittel zu dem genannten Lagerungsschlitten oder von dem genannten Gehäuse weg transportiert werden können, so daß sie bei den Scanvorgängen nicht stören.

8. Gehäuse- und Palettenanordnung zur Verwendung bei nuklearer Abbildung nach einem der Ansprüche 5 bis 7, weiterhin umfassend ein Mittel (92) zum Bewegen der genannten bogenförmigen Palette (54) zwischen einer eingezogenen Stellung und einer Stellung, bei der die genannte bogenförmige Palette leicht zu und von dem genannten Tragmittel transportiert werden kann.

9. Gehäuse- und Palettenanordnung zur Verwendung bei nuklearer Abbildung nach Anspruch 8, wobei das genannte Bewegungsmittel ein Paar teleskopischer Arme (92) aufweist, die schwenkbar mit der genannten Basis (56) in einer im wesentlichen parallelen Weise verbunden sind, wobei mit den genannten teleskopischen Armen ein Mittel (98) zum Ergreifen der genannten bogenförmigen Palette und zum Bewegen derselben zwischen einer Stellung, von der die genannte Palette leicht zu und von dem genannten Tragmittel transportiert werden kann, und einer Stellung, in der die genannte Palette in unmittelbarer Nähe zu dem genannten Cestellständer gelagert werden kann, verbunden ist.

10. Gehäuse- und Palettenanordnung zur Verwendung bei nuklearer Abbildung nach einem der Ansprüche 4 bis 9, wobei das genannte Führungsmittel (12) ein Paar im wesentlichen paralleler Verschiebungsspuren aufweist, die auf einer Gehäusestützplatfform (4) montiert sind, so daß das genannte Gehäuse entlang der genannten Verschiebungsspuren verschoben werden kann.

11. Gehäuse- und Palettenanordnung zur Verwendung bei nuklearer Abbildung nach einem der vorhergehenden Ansprüche, wobei das genannte Gehäuse (2) eine Gehäuseplatte (6) mit einer zweiten Öffnung (20) aufweist, die so darin angeordnet ist, daß die genannte Palette (52) durch die genannte zweite Öffnung passieren kann, wenn das genannte Gehäuse relativ zu der genannten Palette verschoben wird, wobei die genannte Gehäuseplatte (6) lotrecht mit einer Gehäusebasis (8) mit wenigstens einem von dieser verlaufenden Stützfuß (10) verbunden ist, wobei der genannte Rotationsring (24) drehbar auf der genannten Gehäuseplatte koaxial zu der genannten Öffnung angeordnet ist.

12. Gehäuse- und Palettenanordnung zur Verwendung bei nuklearer Abbildung nach Anspruch 11, wobei die genannte Gehäuseplatte (6) lotrecht dazu mit einer Mehrzahl von Nutspurrollen (22) verbunden ist, die mit dem genannten Rotationsring (24) zum rotierenden Führen des genannten Rotationsringes zusammenwirkt, wobei das genannte Mittel (30) zum Drehen des genannten Rotationsringes einen ersten Antriebsmotor (30) aufweist, der drehbar mit einem Stimrädergetriebe (28) verbunden ist, das rotierend mit dem genannten Rotationsring zusammenwirkt, wobei das genannte Stirnrädergetriebe in einem in der genannten Gehäuseplatte angeordneten Schlitz (26) gelagert ist.

13. Gehäuse- und Palettenanordnung zur Verwendung bei nuklearer Abbildung nach einem der vorhergehenden Ansprüche, weiterhin umfassend ein Paar radialer Rückplatten (32), die mit dem genannten Rotationsring (24) verbunden sind, wobei jede der genannten Rückplatten eine Detektormontageplatte (44) beweglich verbunden ist, wobei das genannte Mittel zur radialen Verschiebung der genannten Detektoren ein Schneckengetriebe (38) aufweist, das an jedem Ende drehbar auf einem mit der genannten Rückplatte (32) verbundenen Schneckengetriebelager (40) gelagert ist, wobei das genannte Schneckengetriebe einen beweglich darauf angeordneten Montageblock (42), mit dem die genannte Detektormontageplatte (44) verbunden ist, und einen Antriebsmotor (36) zum Zusammenwirken mit dem genannten Schneckengetriebe aufweist, so daß die genannten Detektoren radial relativ zu dem genannten Ring verschoben werden.

14. Gehäuse- und Palettenanordnung zur Verwendung bei nuklearer Abbildung nach Anspruch 13, wobei jede der genannten Rückplatten (32) weiterhin eine Motorhalterung (34) aufweist, auf der der genannte Antriebsmotor montiert ist, wobei das genannte Mittel zum Verschieben jedes der genannten Detektoren zwischen der genannten radialen Rückplatte (32) und der genannten Detektormontageplatte (44) angeordnet ist.

15. Gehäuse- und Palettenanordnung zur Verwendung bei nuklearer Abbildung nach einem der vorhergehenden Ansprüche, wobei das genannte Mittel zur Erzeugung einer relativen Verschiebungsbewegung zwischen dem genannten Gehäuse und der genannten Palette einen zweiten Antriebsmotor (16) mit einem rotierend mit diesem verbundenen Kettenrad (18), wobei der genannte Antriebsmotor und das Kettenrad mit der genannten Gehäusebasis (8) verbunden sind, und eine Antriebskette (14) aufweist, die verzahnend mit dem genannten Kettenrad (18) zusammenwirkt, wobei die genannte Antriebskette mit jedem Ende der genannten Stützplamorm verbunden ist, so daß bei Aktivierung des genannten zweiten Antriebsmotors das genannte Kettenrad mit der genannten Antriebskette zusammenwirkt, so daß das genannte Gehäuse über ein Paar Verschiebungsspuren (12) verschoben wird, die im wesentlichen parallel zu einer Längsachse des genannten Rotationsrings liegen.

16. Gehäuse- und Palettenanordnung zur Verwendung bei nuklearer Abbildung nach einem der vorhergehenden Ansprüche, wobei das Mittel zur Erzeugung einer relativen Verschiebungsbewegung zwischen dem Gehäuse und der Palette ein Mittel zur lateralen Verschiebung des Gehäuses entlang eine über ihrer Länge abgestützte Verschiebungsspur (12) aufweist.

17. Gehäuseanordnung bei Verwendung bei nuklearer Abbildung, die folgendes umfaßt:
ein Gehäuse (2) mit einem rotierend darauf angeordneten Rotationsring (24), wobei der genannte Rotationsring eine Öffnung definiert, durch die ein Patient während eines Scans passieren kann;
wenigstens einen Detektor (46), der in unmittelbarer Nähe zu dem genannten Rotationsring auf eine allgemein feste Weise mit diesem verbunden ist;
ein Mittel (30) zum Drehen des genannten Rotationsringes (24), so daß der genannte wenigstens eine Detektor (46) gedreht wird, so daß Daten zur Darstellung der Verteilung eines radioaktiv markierten Pharmazeutikums erfaßt werden, das zuvor in den Körper eines Patienten eingespritzt wurde, wobei das Mittel zum Drehen des genannten Ringes wenigstens eine Stellung aufweist, in der sich der Detektor in einer horizontal ausgerichteten Stellung befindet;
ein Mittel (14, 16) zum lateralen Verschieben des genannten Gehäuses (2) relativ zu einer Palette (52, 54) entlang eines Führungsmittels zum Tragen eines Patienten, so daß der genannte wenigstens eine Detektor (46) über den Körper des Patienten passiert und dabei Daten von diametrischen Bereichen des Körpers des Patienten sammelt, die zur Durchführung eines Ganzkörperscans verwendet werden;
ein Mittel (38) zur radialen Verschiebung des genannten wenigstens einen Detektors (46) relativ zu dem genannten Ring, so daß der genannte Detektor relativ zu dem Patienten bewegt werden kann, wodurch eine optimale Positionierung des genannten Detektors während der Durchführung eines Scans erzielt wird.

18. Gehäuseanordnung zur Verwendung bei nuklearer Abbildung nach Anspruch 17, wobei ein Paar im wesentlichen paralleler Verschiebungsspuren (12) auf einer Gehäusetragplatfform (4) montiert ist, so daß das Gehäuse über die genannten Verschiebungsspuren verschoben werden kann, wobei der genannte Rotationsring schrittweise gedreht werden kann.

19. Gehäuseanordnung zur Verwendung bei nuklearer Abbildung nach Anspruch 17 oder Anspruch 18, die zwei entgegengesetzte Detektoren aufweist und bei der das genannte Gehäuse (2) eine Gehäusepiatte (6) mit einer zweiten Öffnung (20) darin aufweist, die so angeordnet ist, daß die Palette durch die genannte zweite Öffnung passieren kann, wenn das genannte Gehäuse relativ zu der genannten Palette verschoben wird, wobei die genannte Gehäuseplatte lotrecht mit einer Gehäusebasis (8) mit wenigstens einem davon weg verlaufenden Stützfuß (10) verbunden ist, wobei der genannte Rotationsring rotierend auf der genannten Gehäuseplatte koaxial zu der genannten Öffnung angeordnet ist.

20. Gehäuseanordnung zur Verwendung bei nuklearer Abbildung nach Anspruch 19, wobei mit der genannten Gehäuseplatte (6) lotrecht eine Mehrzahl von Nutspurrollen (22) verbunden ist, die zu dem genannten Rotationsring zusammenwirkt, um den genannten Ring rotierend zu führen.

21. Gehäuseanordnung zur Verwendung bei nuklearer Abbildung nach Anspruch 19 oder Anspruch 20, wobei das genannte Mittel zum Drehen des genannten Rotationsringes (24) einen ersten Antriebsmotor (30) aufweist, der rotierend mit einem Stirnrädergetriebe (28) verbunden ist, das rotierend mit dem genannten Rotationsring zusammenwirkt, wobei das genannte Stirnrädergetriebe in einem in der Gehäuseplatte angeordneten Schlitz (26) gelagert ist.

22. Gehäuseanordnung zur Verwendung bei nuklearer Abbildung nach einem der Ansprüche 19 bis 21, weiterhin umfassend ein mit dem genannten Rotationsring verbundenes Paar radialer Rückplatten (32), wobei mit jeder der genannten Rückplatten eine Detektormontageplatte (44) beweglich verbunden ist, wobei das genannte Mittel zum radialen Verschieben jedes der genannten Detektoren ein Schneckengetriebe (38) aufweist, das an jedem Ende drehbar auf einem mit der genannten Rückplatte (32) verbundenen Schneckengetriebelager (40) gelagert ist, wobei das genannte Schneckengetriebe einen beweglich darauf angeordneten Montageblock (42), mit dem eine Detektormontageplatte verbunden ist, und einen Antriebsmotor (36) zum Zusammenwirken mit dem genannten Schneckengetriebe aufweist, so daß die genannten Detektoren radial relativ zu dem genannten Ring verschoben werden können.

23. Gehäuseanordnung zur Verwendung bei nuklearer Abbildung nach Anspruch 22, wobei jede der genannten Rückplatten (32) außerdem eine Motorhaltung (34) aufweist, auf der der genannte Antriebsmotor (36) montiert ist, wobei das genannte Mittel zum Verschieben jedes der genannten Detektoren zwischen einer radialen Rückplatte und einer Detektormontageplatte angeordnet ist.

24. Gehäuseanordnung zur Verwendung bei nuklearer Abbildung nach einem der Ansprüche 17 bis 23, wobei das genannte Mittel zur lateralen Verschiebung des genannten Gehäuses einen zweiten Antriebsmotor (16) mit einem rotierend damit verbundenen Kettenrad (18), wobei der genannte Antriebsmotor und das Kettenrad mit der genannten Gehäusebasis (8) verbunden sind, und eine Antriebskette (14) aufweist, die verzahnend mit dem genannten Kettenrad zusammenwirkt, wobei die genannte Antriebskette an jedem Ende so mit der genannten Stützplatfform verbunden ist, daß bei Aktivierung des genannten zweiten Antriebsmotors das genannte Kettenrad mit der genannten Antriebskette zusammenwirkt, so daß das genannte Gehäuse über ein Paar Verschiebungsspuren (12) verschoben wird, die im wesentlichen parallel zu einer Längsachse des genannten Rotationsringes sind.

25. Gehäuseanordnung zur Verwendung bei nuklearer Abbildung nach einem der Ansprüche 17 bis 24, wobei das Mittel zur Erzeugung der relativen Verschiebung zwischen dem Gehäuse und der Palette ein Mittel zur lateralen Verschiebung des Gehäuses über eine Verschiebungsspur (12) aufweist.

## Revendications

1. Ensemble de portique et de palette utilisé en imagerie nucléaire, comportant:
un portique (2) présentant un anneau de rotation (24) définissant une ouverture à travers laquelle un patient peut passer lors d'une scanographie, ledit anneau de rotation définissant également un axe longitudinal lorsqu'il est translaté latéralement;
deux détecteurs (46) placés diamétralement, reliés de façon rigide audit anneau et à proximité de celui-ci;
un moyen de translation radiale des détecteurs (46) sur l'anneau (24) de manière à ce que les détecteurs puissent se déplacer radialement par rapport à un patient et par rapport à l'axe longitudinal;
un moyen (30) de rotation dudit anneau de manière à ce que lesdits détecteurs soient tournés, acquérant ainsi des données utilisées pour tracer la distribution d'un pharmaceutique radioactivement marqué précédemment injecté dans le corps d'un patient, le moyen de rotation dudit anneau présentant au moins une position dans laquelle les détecteurs placés sont dans un plan horizontal;
une première palette (52) supportée à chaque extrémité par un moyen de support (110,112) de manière à ce que ladite première palette soit sensiblement coaxiale audit axe longitudinal, ladite palette étant positionnée de manière à ce qu'elle soit aisément accessible pour qu'un patient monte et descende de celle-ci;
un moyen (14,16) pour translater latéralement ledit portique (2) par rapport à ladite première palette (52) le long d'un moyen de guidage de manière à ce que lesdits détecteurs (46) passent par dessus le corps du patient supporté sur ladite première palette (52), acquérant ainsi des données des zones diamétrales du corps du patient utilisées pour tracer la distribution du pharmaceutique radioactivement marqué précédemment injecté dans le corps du patient, le corps du patient passant au moins partiellement dans ladite ouverture lorsque ledit portique et ladite première palette sont translatés l'un par rapport à l'autre.

2. Ensemble de portique et de palette utilisé en imagerie nucléaire selon la revendication 1, dans lequel la première palette (52) est positionnée de manière à ce qu'une extrémité soit adjacente à au moins un détecteur (46) lorsque ledit portique est dans une position de manière à ce que ledit au moins un détecteur puisse acquérir des données de la région de la tête d'un patient lorsqu'il est allongé sur ladite palette, les données étant utilisées pour tracer la distribution d'un pharmaceutique radioactivement marqué précédemment injecté dans le corps du patient.

3. Ensemble de portique et de palette utilisé en imagerie nucléaire selon la revendication 2, dans lequel ledit moyen de support (110,112) est verticalement réglable de manière à ce que la relation spatiale entre ladite palette (52) et ledit au moins un détecteur (46) peut être réglée déplaçant ainsi un patient supporté par ladite palette en le rapprochant ou en l'éloignant davantage dudit au moins un détecteur pour réaliser le positionnement désiré du patient afin de réaliser une procédure d'imagerie particulière, ledit anneau de rotation (24) pouvant être tourné de façon incrémentielle.

4. Ensemble de portique et de palette utilisé en imagerie nucléaire selon l'une quelconque des revendications 1 à 3, dans lequel ladite première palette (52) est sensiblement rectangulaire et plate et utilisée pour la scanographie du corps total de sorte que, lors de l'utilisation, ledit portique (2) est translaté latéralement par rapport à ladite première palette (52) le long d'un moyen de guidage (12) provoquant le passage longitudinal de ladite première palette au travers de ladite ouverture, ladite première palette étant supportée de manière détachable à chaque extrémité audit moyen de support.

5. Ensemble de portique et de palette utilisé en imagerie nucléaire selon la revendication 4, dans lequel ladite première palette (52) est interchangeable avec une deuxième palette (54) qui est sensiblement arquée et utilisée pour une scanographie SPECT⁽¹⁾ rotative de sorte que ledit au moins un détecteur soit positionné au-dessus d'une zone d'intérêt du corps d'un patient ensuite tourné acquérant ainsi une quantité suffisante d'informations pour tracer la distribution d'un pharmaceutique marqué radioactivement précédemment introduit dans le corps d'un patient, ladite deuxième palette (54) étant détachable et supportée par ledit moyen de support (110,112), lesdites première et deuxième palettes étant emmagasinables sur un chariot d'emmagasinage (50) positionné à proximité dudit portique (2), ledit chariot d'emmagasinage présentant un cadre (64) monté au-dessus d'un bâti (56) et adapté pour retenir ladite première palette, ledit cadre présentant un composant de cadre (68) étirable qui peut être positionné de manière à ce que ladite première palette puisse être facilement retirée de là ou placée dessus.

6. Ensemble de portique et de palette utilisé en imagerie nucléaire selon la revendication 5, dans lequel ledit chariot d'emmagasinage (50) comporte en outre une paire de montants de support (64) de cadre s'étendant dudit bâti (56), ledit composant de cadre (68) étirable étant monté de façon pivotante auxdits montants, chacun desdits montants présentant un contrefort en saillie relié à celui-ci pour empêcher ledit cadre de pivoter au-delà de l'horizontal dans un sens, ledit composant de cadre étirable présentant quatre chevilles de retenue de palette (70) s'étendant de là pour retenir ladite première palette (52), deux desdites chevilles ayant chacune une ouverture à l'intérieur, dans laquelle un arrêt (74) chargé par ressort est inséré pour exercer une pression contre ladite première palette lorsqu'elle est positionnée sur ledit cadre, maintenant ainsi la première palette fermement à l'intérieur dudit cadre.

7. Ensemble de portique et de palette utilisé en imagerie nucléaire selon la revendication 5 ou la revendication 6, dans lequel ledit composant de cadre (68) étirable est monté de façon coulissante à l'intérieur d'un composant de cadre externe de manière à ce que ledit composant de cadre étirable puisse être positionné entre une position d'emmagasinage et une position où ladite première palette (52) puisse être facilement retirée de là ou placée sur celui-ci, ledit chariot d'emmagasinage étant positionné de façon déplaçable et sensiblement parallèle audit axe longitudinal de manière à ce que ledit chariot d'emmagasinage (50) puisse être positionné près dudit portique (2) pour transférer lesdites palettes dudit chariot d'emmagasinage audit moyen de support (110,112) ou dudit moyen de support audit chariot d'emmagasinage, ou loin dudit portique afin qu'il n'interfère pas avec les procédures de scanographie.

8. Ensemble de portique et de palette utilisé en imagerie nucléaire selon l'une quelconque des revendications 5 à 7, comportant en outre un moyen (92) de déplacement de ladite palette (54) arquée entre une position retirée et une position où ladite palette arquée peut être facilement transférée audit moyen de support ou transférée dudit moyen de support.

9. Ensemble de portique et de palette utilisé en imagerie nucléaire selon la revendication 8, dans lequel ledit moyen de déplacement comporte une paire de bras (92) télescopiques reliés de façon pivotante audit bâti (56) d'une façon sensiblement parallèle, sur lesdits bras télescopiques est relié un moyen (98) pour saisir ladite palette arquée et la déplacer entre une position où ladite palette peut être facilement transférée audit moyen de support ou transférée dudit moyen de support, et une position où ladite palette est emmagasinée à proximité desdits montants de support de cadre (64).

10. Ensemble de portique et de palette utilisé en imagerie nucléaire selon l'une quelconque des revendications 4 à 9, dans lequel ledit moyen de guide (12) comporte une paire de glissières de translation sensiblement parallèles montées sur une plate-forme de support de portique (4) de manière à ce que ledit portique puisse être translaté le long desdites glissières de translation.

11. Ensemble de portique et de palette utilisé en imagerie nucléaire selon l'une quelconque des revendications précédentes, dans lequel ledit portique (2) comporte une plaque de portique (6) présentant une deuxième ouverture (20) disposée à l'intérieur de manière à ce que ladite palette (52) puisse passer au travers de ladite deuxième ouverture lorsque ledit portique est déplacé de façon translationnelle par rapport à ladite palette, ladite plaque de portique (6) étant reliée de façon perpendiculaire à un bâti de portique (8) présentant au moins une jambe de support (10) s'étendant de là, ledit anneau de rotation (24) étant disposé de façon rotative, sur ladite plaque de portique, coaxial à ladite ouverture.

12. Ensemble de portique et de palette utilisé en imagerie nucléaire selon la revendication 11, dans lequel sur ladite plaque de portique (6), et perpendiculairement à celle-ci, est reliée une pluralité de rouleaux de glissière de rainure (22) adaptés pour coopérer avec ledit anneau de rotation (24) pour guider de façon rotative ledit anneau de rotation, ledit moyen (30) pour tourner ledit anneau de rotation comportant un premier moteur d'entraînement (30) relié de façon rotative à une roue à dents droites (28) qui coopère de façon rotative avec ledit anneau de rotation, ladite roue à dents droites étant supportée dans une fente (26) disposée dans ladite plaque de portique.

13. Ensemble de portique et de palette utilisé en imagerie nucléaire selon l'une quelconque des revendications précédentes, comportant en outre une paire de plaques arrières (32) radiales reliées audit anneau de rotation (24), sur chacune desdites plaques arrières est reliée de façon déplaçable une plaque d'assemblage de détecteur (44), ledit moyen de translation radiale desdits détecteurs comportant un engrenage à vis sans fin (38) relié au moyen d'un tourillon, à chaque extrémité, à un support d'engrenage à vis sans fin (40) relié à ladite plaque arrière (32), ledit engrenage à vis sans fin présentant un bloc d'assemblage (42) disposé de façon déplaçable sur celui-ci sur lequel ladite plaque d'assemblage de détecteur (44) est reliée, et un moteur d'entraînement (36) pour coopérer avec ledit engrenage à vis sans fin de manière à ce que lesdits détecteurs soient translatés radialement par rapport audit anneau.

14. Ensemble de portique et de palette utilisé en imagerie nucléaire selon la revendication 13, dans lequel chacune desdites plaques arrières (32) comporte en outre un bâti de moteur (34) sur lequel ledit moteur d'entraînement est monté, ledit moyen de translation de chacun desdits détecteurs étant disposé entre ladite plaque arrière (32) radiale et ladite plaque d'assemblage de détecteur (44).

15. Ensemble de portique et de palette utilisé en imagerie nucléaire selon l'une quelconque des revendications précédentes, dans lequel ledit moyen de production d'un déplacement translationnel relatif entre ledit portique et ladite palette comporte un deuxième moteur d'entraînement (16) ayant un pignon (18) relié de façon rotative à celuici, ledit moteur d'entraînement et le pignon reliés audit bâti de portique (8), une chaîne d'entraînement (14) qui coopère de façon engrenable avec ledit pignon (18), ladite chaîne d'entraînement reliée, à chaque extrémité, à ladite plate-forme de support de manière à ce que lorsque ledit deuxième moteur d'entraînement est activé, ledit pignon coopère avec ladite chaîne d'entraînement translatant ainsi le portique le long d'une paire de glissières de translation (12) qui sont sensiblement parallèles à un axe longitudinal dudit axe de rotation.

16. Ensemble de portique et de palette utilisé en imagerie nucléaire selon l'une quelconque des revendications précédentes, dans lequel le moyen de production de déplacement translationnel relatif entre le portique et la palette comporte un moyen pour translater latéralement le portique le long d'une glissière translationnelle (12) supportée sur sa longueur.

17. Ensemble de portique utilisé en imagerie nucléaire, comportant:
un portique (2) présentant un anneau de rotation (24) disposé de façon rotative sur celui-ci, ledit anneau de rotation définissant une ouverture à travers de laquelle un patient peut passer durant une scanographie;
au moins un détecteur (46) relié d'une manière généralement rigide et audit anneau de rotation et à proximité de celui-ci;
un moyen (30) de tourner ledit anneau de rotation (24) de manière à ce que ledit au moins un détecteur (46) soit tourné, acquérant ainsi des données utilisées pour tracer la distribution d'un pharmaceutique radioactivement marqué précédemment injecté dans le corps d'un patient, le moyen de rotation de ladite bague présentant au moins une position dans laquelle le détecteur est dans une position de face horizontale;
un moyen (14,16) pour translater latéralement ledit portique (2) par rapport à une palette (52,54) le long d'un moyen de guidage pour supporter un patient, de sorte que ledit au moins un détecteur (46) passe au-dessus du corps du patient, acquérant des données des surfaces diamétrales du corps du patient utilisé pour réaliser une scanographie du corps total;
un moyen (38) pour translater radialement ledit au moins un détecteur (46) par rapport audit anneau de manière à ce que ledit détecteur puisse être déplacé par rapport au patient, fournissant ainsi un positionnement optimal dudit détecteur lors de la scanographie.

18. Ensemble de portique utilisé en imagerie nucléaire selon la revendication 17, dans lequel une paire de glissières (12) de translation sensiblement parallèles sont montées sur une plate-forme de support de portique (4) de manière à ce que ledit portique puisse être translaté le long desdites glissières de translation, ledit anneau de rotation étant apte à être tourné de façon incrémentielle.

19. Ensemble de portique utilisé en imagerie nucléaire selon 17 ou la revendication 18, comportant deux détecteurs opposés et dans lequel ledit portique (2) comporte une plaque (6) de portique présentant une deuxième ouverture (20) disposée à l'intérieur de manière à ce que la palette puisse passer au travers de ladite deuxième ouverture lorsque ledit portique est déplacé de façon translationnelle par rapport à ladite palette, ladite plaque de portique étant reliée perpendiculairement à un bâti de portique (8) présentant au moins une jambe de support (10) s'étendant de là, ledit anneau de rotation étant disposé de façon rotative, sur ladite plaque de portique, coaxial à ladite ouverture.

20. Ensemble de portique utilisé en imagerie nucléaire selon la revendication 19, dans lequel sur ladite plaque de portique (6) est perpendiculairement relié une pluralité de rouleaux de glissières de rainure (22) adaptés pour coopérer avec ledit anneau de rotation (24) pour guider de façon rotative ledit anneau.

21. Ensemble de portique utilisé en imagerie nucléaire selon la revendication 19 ou la revendication 20, dans lequel ledit moyen de rotation dudit anneau de rotation (24) comporte un premier moteur d'entraînement (30) relié de façon rotative à une roue à dents droites (28) qui coopère de façon rotative avec ledit anneau de rotation, ladite roue à dents droites étant supportée dans une fente (26) disposée dans ladite plaque de portique.

22. Ensemble de portique utilisé en imagerie nucléaire selon l'une quelconque des revendications 19 à 21, comportant en outre une paire de plaques (32) arrières radiales reliées audit anneau de rotation, sur chacune desdites plaques arrières est relié de façon déplaçable une plaque d'assemblage de détecteur (44), ledit moyen pour translater de façon radiale chacun desdits détecteurs comportant un engrenage à vis sans fin (38) relié au moyen d'un tourillon, à chaque extrémité, à un support d'engrenage à vis sans fin (40) relié à ladite plaque arrière (32), ledit engrenage à vis sans fin présentant un bloc d'assemblage (42) disposé de façon déplaçable sur celui-ci sur lequel une plaque d'assemblage de détecteur est reliée, et un moteur d'entraînement (36) pour coopérer avec ledit engrenage à vis sans fin de manière à ce que lesdits détecteurs soient translatés radialement par rapport audit anneau.

23. Ensemble de portique utilisé en imagerie nucléaire selon la revendication 22, dans lequel chacune desdites plaques arrières (32) comporte en outre un bâti de moteur (34) sur lequel ledit moteur d'entraînement (36) est monté, ledit moyen de translation de chacun desdits détecteurs étant disposé entre une plaque arrière radiale et une plaque d'assemblage de détecteur.

24. Ensemble de portique utilisé en imagerie nucléaire selon l'une quelconque des revendications 17 à 23, dans lequel ledit moyen de translation latérale dudit portique comporte un deuxième moteur d'entraînement (16) ayant un pignon (18) relié de façon rotative à celui-ci, ledit moteur d'entraînement et le pignon étant reliés audit bâti de portique (8), une chaîne d'entraînement (14) qui coopère de façon engrenable avec ledit pignon, ladite chaîne d'entraînement reliée, à chaque extrémité, à ladite plate-forme de support de manière à ce que lorsque ledit deuxième moteur d'entraînement est activé, ledit pignon coopère avec ladite chaîne d'entraînement, translatant ainsi ledit portique le long d'une paire de glissières de translation (12) qui sont sensiblement parallèles à un axe longitudinal dudit anneau de rotation.

25. Ensemble de portique utilisé en imagerie nucléaire selon l'une quelconque des revendications 17 à 24, dans lequel le moyen de production de déplacement translationnel relatif entre le portique et la palette comporte un moyen de translation latérale du portique le long d'une glissière translationnelle (12).
